# EUROPÄISCHE PATENTSCHRIFT

(11) **EP 2 071 037 B1**
(45) Veröffentlichungstag und Bekanntmachung des Hinweises auf die Patenterteilung: **20.07.2016**
(21) Anmeldenummer: 09155622.5
(22) Anmeldetag: 12.08.1999
(51) Int. Cl.: C12Q 1/68, A61B 5/15

(54) **Gefäß zur Entnahme von Blut**
Vessel for blood sampling
Recipient servant a un prelevement de sang

(30) Priorität: 12.08.1998 DE 19836559
(43) Veröffentlichungstag der Anmeldung: 17.06.2009
(62) Teilanmeldung aus: 99941580.5
(73) Patentinhaber: PreAnalytiX GmbH, 8634 Hombrechtikon (CH)
(72) Erfinder: Helftenbein, Elke, 70597, Stuttgart (DE)
(74) Vertreter: Vossius & Partner Patentanwälte Rechtsanwälte mbB

(56) Entgegenhaltungen:
- EP-A- 0 554 034
- EP-A- 0 818 542
- WO-A-97/05248
- DE-A1- 19 702 907
- LOZANO M.E. ET AL.: "A simple nucleic acid amplification assay for the rapid detection of Junín virus in whole blood samples" VIRUS RESEARCH, Bd. 27, 1993, Seiten 37-53, XP002900733
- MACDONALD R.J. ET AL.: "ISOLATION OF RNA USING GUANIDINIUM SALTS" METHODS IN ENZYMOLOGY, ACADEMIC PRESS INC, SAN DIEGO, CA, US, Bd. 152, 1. Januar 1987 (1987-01-01), Seiten 219-227, XP000886523 ISSN: 0076-6879

## Beschreibung

Die vorliegende Erfindung betrifft ein Gefäß zur Entnahme von Blut, wobei das entnommene Blut insbesondere zur Nukleinsäürestabilisierung und -analytik eingesetzt werden soll.

Während der Abnahme wird Blut herkömmlicherweise in Gefäßen aufgefangen, die bereits Antikoagulantien wie z.B. Heparin, Citrat oder EDTA enthalten. So wird verhindert, daß das Blut gerinnt. So gewonnene Blutproben lassen sich längere Zeit bei geeigneten Temperaturen lagern. Diese Art der Blutgewinnung hat jedoch erhebliche Nachteile, wenn Nukleinsäuren, wie z.B. (m)RNA oder DNA analysiert werden sollen. Für solche Zwecke sollten die Nukleinsäuren, die in der Probe enthalten sind, am besten bereits im Moment der Abnahme stabilisiert werden, d.h. es sollte der Abbau der vorhandenen Nukleinsäuren, aber auch die Neusynthese von mRNA verhindert werden.

Dieses Ziel der stabilen Lagerung der im Probenmaterial enthaltenen Nukleinsäuren vom Moment der Abnahme an ist bei der Lagerung von Blut aus folgenden Gründen bis jetzt praktisch nicht zu bewerkstelligen:
Zellen enthalten Nukleasen, Enzyme, die Nukleinsäuren zerstören, sobald sie mit ihren Substraten (RNA, DNA) in Kontakt kommen. Die Wirkung zellulärer und extrazellulärer Nukleasen ist normalerweise unter physiologischer Kontrolle, solange die Zellen in ihrer normalen Umgebung sind. Die Entnahme von Blut führt zu mehr oder weniger starken Veränderungen der in den Zellen enthaltenen Nukleinsäuren. Nukleasen werden dann innerhalb der Zellen und/oder durch die Lyse von Zellen nach außen freigesetzt. Außerdem werden Nukleinsäuren mehr oder weniger stark synthetisiert. Gerade die Langzeitlagerung von Blut führt zur Alterung und Zerstörung der Zellen.

Ein weiteres Problem bei der Langzeitlagerüng von Blutproben, die nach herkömmlichen Abnahmeverfahren gewonnen wurden, ist die starke Veränderung des Probenmaterials. Solche Veränderungen wie z.B. starke Lyse von Zellen, können dazu führen, daß die Standardverfahren der Nukleinsäureisolierung nicht mehr mit befriedigender Effizienz funktionieren.

Abgesehen von den Problemen einer stabilen Lagerung von Nukleinsäuren, die im Probenmaterial enthalten sind, ergeben sich beim herkömmlichen Verfahren der Blutentnahme weitere Schwierigkeiten. Die herkömmlichen Antikoagulantien werden oft bei der Nukleinsäureisolierung nicht mit genügender Effizienz abgetrennt und stören bei der nachfolgenden Nukleinsäureanalytik, wie z.B. bei der Amplifikation mittels PCR (Polymerase Chain Reaction). Heparin ist z.B. ein allgemein bekannter Inhibitor der PCR.

Schließlich ergibt sich bei der quantitativen Nukleinsäureanalytik die Frage, wie das gesamte Verfahren von der Probennahme bis hin zur Nukleinsäuremessung unter standardisierten Bedingungen kontrolliert werden kann. Idealerweise sollte dem Probenmaterial bereits bei der Entnahme eine bezüglich Menge und Qualität definierte Standardnukleinsäure zugesetzt werden, die dem gesamten Prozeß der Probennahme und Bestimmung unterzogen wird. Auch dies ist mit den herkömmlichen Abnahmesystemen nicht zu bewerkstelligen.

Ein weiterer Nachteil der konventionellen Blutentnahme ist die Gefahr der Übertragung von infektiösem Material, da bisher für die Nukleinsäureisolierung manuelle Verfahrensschritte notwendig sind. Ein Kontakt mit potentiell infektiösen Erregern kann nicht ausgeschlossen werden.

In der Literatur ist ein Verfahren beschrieben, bei dem die Blutprobe unmittelbar nach der Abnahme vom Patienten mit Guanidiniumsalz vermischt wird (EP 0 818 542 A1). Bei diesem Verfahren liegt das Guanidiniumsalz in Pulverform vor, um somit die höhere Stabilität des Guanidiniumsalzes zu nutzen. Dieses Verfahren besitzt jedoch gravierende Nachteile, da sich das Salz z.B. zunächst in dem zugegebenen Blut lösen muß. Der Lösungsvorgang ist insbesondere temperaturabhängig und aufgrund des verwendeten, undurchsichtigen Probenmaterials nicht zu kontrollieren. Die Verwendung eines entspechenden Produktes für diagnostisch-medizinische Zwecke ist somit äußerst problematisch.

Ferner sind Nukleasen äußerst aktive Enzyme, die nur unter extrem denaturierenden Bedingungen zu inhibieren sind. Die Denaturierung ist abhängig von der Konzentration des Guanidiniumsalzes in Lösung. Eine inhibierende Konzentration von Guanidiniumsalz in Lösung ist bei dem zitierten Verfahren nicht von Anfang an gegeben. Es kommt also zum unkontrollierten Abbau von Nukleinsäuren während des Lösungsvorgangs. Bei diesem Verfahren wird außerdem auf den Zusatz von reduzierenden Agenzien verzichtet, ohne die eine wirksame Inhibition - insbesondere von RNasen - nicht gewährleistet ist (siehe Beispiel Nr. 5).

Die so hergestellte Probe kann außerdem nicht direkt für die weitere Nukleinsäureisolierung an Glasoberflächen verwendet werden. Die Verwendung von Guanidiniumsalzpulver erlaubt außerdem nicht den Zusatz von internen Nukleinsäurestandards. Solche Standards sind zur Verfahrenskontrolle und genauen Quantifizierung unerläßlich.

Die Verwendung einer Lösung enthaltende 8 M Guanidinium Isothiocyanat, 1 % Sarkosyl und 50 mM Natriumcitrat zur Isolierung von RNA zur Detektion von Junin Virus in Blutproben wird in Lozano et al. (Virus Research 27(1993):37-53) beschrieben. Hier geht es allerdings nicht um die Stabilisierung der Nukleinsäuren aus der Blutprobe per se, sondern um die Stabilisierung der möglicherweise vorhandenen viralen RNA. Die Verwendung einer Guanidiniumthiocyanat Lösung zur Denaturierung von Proteinen aus Gewebeproben wird in MacDonald et al. beschrieben (Methods in Enzymology 152(1987):219-227), wobei hier weder allgemein auf die Langzeitstabilisierung von Nukleinsäuren noch speziell auf Blutproben bezug genommen wird.

Der vorliegenden Erfindung lag das technische Problem zugrunde, ein Gefäß zur Blutentnahme anzugeben, das die Nachteile aus dem Stand der Technik nicht aufweist. Insbesondere soll die mit dem Gefäß entnommene Probe direkt den gängigen Nukleinsäureanalyseverfahren zugeführt werden können, ohne weitere Probenaufbereitungsschritte durchführen zu müssen.

Dieses Problem wird erfindungsgemäß gelöst durch ein Gefäß zur Entnahme von Blut, welches im zur Blutaufnahme vorgesehenen Raum einen Unterdruck aufweist, enthaltend eine wäßrige Lösung mit folgenden Bestandteilen:
- ein Guanidiniumsalz;
- eine Puffersubstanz; und
- ein Detergenz.

Das erfindungsgemäße Gefäß besitzt folgende Vorteile: 1. Das Blut wird bereits im Moment der Abnahme lysiert, indem das Abnahmegefäß bereits eine Nukleinsäure-stabilisierende Substanz in Lösung enthält. 2. Die Nukleinsäure-stabilisierende Substanz ist so zusammengesetzt, daß das Probenmaterial, insbesondere die darin enthaltenen Nukleinsäuren unmittelbar nach Kontakt mit der Lösung stabilisiert werden. 3. Die stabilisierte Probe muß, im Gegensatz zu allen bisher üblichen Abnahmesystemen wie EDTA- oder heparinhaltigen Abnahmegefäßen, nicht länger als infektiöses Material gehandhabt werden. 4.Die Nukleinsäure-stabilisierende Substanz ist so zusammengesetzt, daß das Probenmaterial direkt in nachfolgenden Isolierungsverfahren verwendet werden kann. 5. Die Nukleinsäure-stabilisierende Substanz kann bei der nachfolgenden Isolierung so effizient abgetrennt werden, daß eine Hemmung der PCR nicht auftritt. 6. Der Nukleinsäure-stabilisierenden Substanz kann ein interner Standard zugesetzt werden. Dieser erlaubt die Kontrolle des gesamten Verfahrens von der Probenentnahme bis hin zur Nükleinsäuredetektion.

Das in Punkt 1 genannte Abnahmegefäß besteht aus einem herkömmlichen Blutentnahmegefäß (Röhrchen), in das ein definiertes Volumen einer Nukleinsäure-stabilisierenden Substanz gegeben wird. Das Röhrchen wird anschließend mit einem definierten Unterdruck versehen, der garantiert, daß nur ein bestimmtes Volumen Blut während der Abnahme zufließen kann. Das Röhrchen kann mit konventionellen Methoden der Blutabnahme gehandhabt werden. Die in dem Röhrchen enthaltene Lösung enthält in ihrer speziellen bevorzugten Ausführungsform folgende Reagenzien: Guanidiniumthiocyanat, Triton-X-100, Dithiothreitol und ein geeignetes Puffersystem wie z.B. Citrat, Tris oder Hepes. In der beschriebenen Zusammensetzung ist die Lösung kompatibel mit dem Vakuumröhrchen. Diese Lösung kann problemlos in dem Vakuumröhrchen gelagert werden, ohne daß es zu einer Beeinträchtigung der gewünschten stabilisierenden Funktion kommt Das gesamte System ist insbesondere für den Blutspender problemlos und sicher bei der Probennahme.

Die Lösung, enthaltend das Guanidiniumsalz, die Puffersubstanz, das Reduktionsmittel und/oder das Detergenz ist lagerungsstabil und verwandelt das zugeführte, frisch entnommene Blut in ein Material, das ebenfalls lagerungsstabil ist und das den gängigen Nukleinsäureanalysekits (wie z.B. von Roche oder Qiagen) unmittelbar zugeführt werden kann.

Als Guanidiniumsalz sind Guanidiniumthiocyanat und/oder Guanidiniumchlorid bevorzugt.

Vorzugsweise liegt das Guanidiniumsalz in einer Konzentration von 2,0 bis 8,0 M vor. Als Puffersubstanz ist Tris oder Citrat bevorzugt, wobei der exakte pH vorzugsweise mit HCl eingestellt wird. Weitere mögliche Puffer sind jedoch HEPES, MOPS, Citrat- und Phosphatpuffer, wie z.B. PBS.

Die Pufferkonzentration liegt vorzugsweise zwischen 10 und 300 mM, besonders bevorzugt zwischen 10 und 100 mM.

Als Detergenz ist Triton-X-100 bevorzugt. Weitere mögliche Detergenzien sind NP-40, Tween 20, Polydocanol oder andere Detergenzien.

Die Detergenzkonzentration liegt vorzugsweise bei 5 bis 30 % (w/v), besonders bevorzugt bei 10 bis 20 % (w/v).

Als Reduktionsmittel ist DTT bevorzugt, wobei jedoch auch β-Mercaptoethanol, TCEP(Tris(2-carboxyethyl)phosphin) oder andere Reduktionsmittel einsetzbar sind.

Die bevorzugte Konzentration des Reduktionsmittels liegt bei 0,1 bis 10 % (w/v); besonders bevorzugt sind 0,5 bis 2 % (w/v).

Der pH der Lösung liegt vorzugsweise bei 3,0 bis 9,0, besonders bevorzugt bei 4,0 bis 7,5, besonders bevorzugt bei 5 bis 6.
Der pH der Lösung wird insbesondere so gewählt, daß sich nach Zugabe des Probenmaterials ein pH-Wert im Bereich von 5,0 bis 7,6 einstellt. Besonders bevorzugt ist ein pH zwischen 6,3 und 6,9 (siehe Beispiel Nr. 8)
Eine besonders bevorzugte Lösung enthält vorzugsweise 4 M Guanidiniumthiocyanat, 45 mM Tris/HCl, 18, vorzugsweise 15 % (w/v) Triton-X-100, 0,8% (w/v) DTT und besitzt einen pH von 6,0.

Das Volumen zur Aufnahme der Blutprobe weist einen Unterdruck auf, der so eingestellt werden kann, das ein vorab bestimmtes Blutvolumen in das Gefäß eingesaugt wird, nachdem ein Blutgefäß angestochen wurde. Entsprechend evakuierte Gefäße sind auf dem Markt erhältlich. Das Gefäß, enthaltend das entnommene Blut, kann dann sofort der weiteren Analytik zugeführt werden oder aber für einen längeren Zeitraum (bis mehrere Tage) ohne Nachteile für die Qualität der Probe aufbewahrt werden.

Bei dem erfindungsgemäßen Verfahren wird das frisch entnommene Blut direkt in dem Blutentnahmegefäß mit der oben beschriebenen Lösung in Kontakt gebracht, so daß sofort sämtliche Vorgänge, die das Nukleinsäuremuster der Probe verändern können, gestoppt werden. Die später ermittelten Daten hinsichtlich der nachgewiesenen Nukleinsäuren stellen daher sehr genau den Ist-Zustand zum Zeitpunkt der Blutentnahme dar, sowohl hinsichtlich der Mengen als auch der Arten der Nukleinsäuren.

Vorzugsweise entspricht die entnommene Blutmenge dem 0,1- bis 4-fachen der in dem Gefäß vorgelegten Lösung. Letztere beträgt vorzugsweise 0,5 bis 5,0 ml. Somit liegt die Endkonzentration an Guanidiniumsalz nach Blutzusatz bei 1,0 bis 5 M, vorzugsweise bei 1 bis 3 M, besonders bevorzugt sind 2-3 M (siehe Bsp. 7).

Das erfindungsgemäße Gefäß wird vorzugsweise dann zur Blutentnahme eingesetzt, wenn die Blutprobe für die Nukleinsäureanalytik verwendet werden soll.

Die Verwendung o.g. Lösung als Bestandteil des beschriebenen Abnahmesystems garantiert allein die sofortige Lyse der Zellen und simultane Stabilisierung der Probe durch unmittelbare Inaktivierung der Nukleasen. Überraschenderweise kann die so gewonnene Blutprobe selbst bei Raumtemperatur oder höher über mehrere Tage gelagert werden. Das Abnahmesystem gewährleistet außerdem eine kontaminationssichere und nicht-infektiöse Handhabung von der Probennahme über die Nuleinsäureisolierung bis hin zur Analytik. Bei den herkömmlichen Verfahren der Nukleinsäureisolierung sind bisher immer zusätzliche Handhabungsschritte (wie die Überführung der entnommenen Blutprobe in die Reagenzien zur Nukleinsäureisolierung usw.) notwendig, die mit einem zusätzlichen Infektionsrisiko verbunden sind.

Die mit dem Blutentnahmesystem gewonnene Probe ist kompatibel mit allen gängigen Standardverfahren der Nukleinsäureisolierung. In diesem Zusammenhang besonders hervorzuheben sind Verfahren, die auf der Bindung von Nukleinsäuren an Glasoberflächen basieren, aber auch sequenzspezifisches Binden an komplementäre Nukleinsäure und Lösungsmittel-basierende Extraktionverfahren.

Die beschriebene Erfindung besteht somit aus einem Blutentnahmesystem, das so konzipiert ist, daß folgende Bedingungen erfüllt werden: 1. Kontrollierte Probenentnahme und gleichzeitige Stabilisierung der im Probenmaterial enthaltenen Nukleinsäuren (DNA, RNA). 2. Probenentnahme, bei der auf den Einsatz von Antikoagulantien vollkommen verzichtet werden kann. 3. Die mit dem beschriebenen Blutentnahmesystem gewonnene Probe kann universell in allen bekannten Nukleinsäure-Isolierungssystemen eingesetzt werden. 4. Das Blutentnahmesystem ist lagerungsstabil.

Zusätzlich wurde überraschenderweise festgestellt, daß die mit dem beschriebenen Abnahmesystem gewonnene Probe in dem Gefäß über längere Zeit ohne Degradation der Nukleinsäuren lagerfähig ist (siehe Beispiele 2, 3, 7, 8).

Die folgenden Beispiele erläutern die Erfindung.

### Beispiele 1:

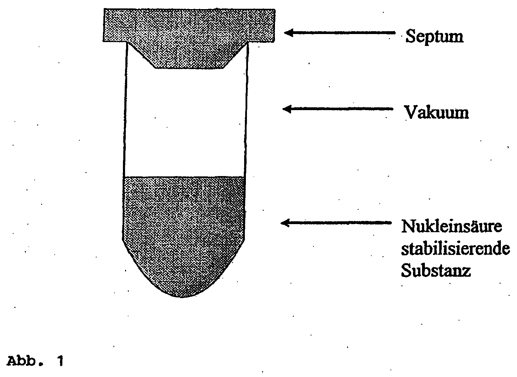

Das Blutentnahmesystem kann in einer bevorzugten Ausführungsform folgendermaßen zusammengesetzt sein (s. Abb. 1): Ein Röhrchen wird mit einem definierten Volumen der Nukleinsäure-stabilisierenden Substanz gefüllt, mit einem definierten Vakuum versehen und mit einem Septum verschlossen. Das Septum ist so konstruiert, daß es mit dem gängigen Probenentnahmezubehör (Kanüle usw.) kompatibel ist. Im vorliegenden Beispiel wurden 2,2 ml Reagenz vorgelegt und das Vakuum war so eingestellt, daß bei der Probennahme exakt 2,2 ml Blut zufließen konnten. Die im zufließenden Blutstrom enthaltenen Nukleinsäuren wurden unmittelbar in eine stabile Form überführt.

Allgemeine Vorbemerkung zu den nachfolgenden Beispielen.

Bei allen nachfolgend beschriebenen Beispielen hatte die Nukleinsäure-stabilisierende Substanz (N-sS), falls nicht anders angegeben, folgende Zusammensetzung: 45 mM Tris, 5 M Guanidiniumthiocyanat (GTC), 0,8% (w/v) Dithiothreitol (DTT), 18% (w/v) Triton-X-100, pH 6,0.

In allen beschriebenen Beispielen wurde die Nukleinsäure-stabilisierende Substanz, falls nicht anders angegeben, mit der Probe im Verhältnis 1 zu 1 gemischt (1 Volumen N-sS plus 1 Volumen Probenmaterial). Eine geringere Konzentration von N-sS, z.B. 1 Volumen N-sS plus 5 Volumen Probe, könnte zur Degradation von RNA führen.

Für alle Beispiele wurde Blut dadurch stabilisiert, daß es unmittelbar bei der Entnahme in das mit N-sS versetzte Röhrchen gegeben wurde.

### Beispiel 2:

Stabilität von Nukleinsäure nach Mischung von Probenmaterial und N-sS.
Isolierung von RNA und DNA vom Probenlysat mit silikaderivatisierten Oberflächen.

### Material und Methode:

Das Probenmaterial für die DNA- und RNA-Isolierung wurde unmittelbar nach der Entnahme, nach Lagerung für 6 Tage bei 4°C und nach Lagerung für 1 Monat bei -20°C verwendet.

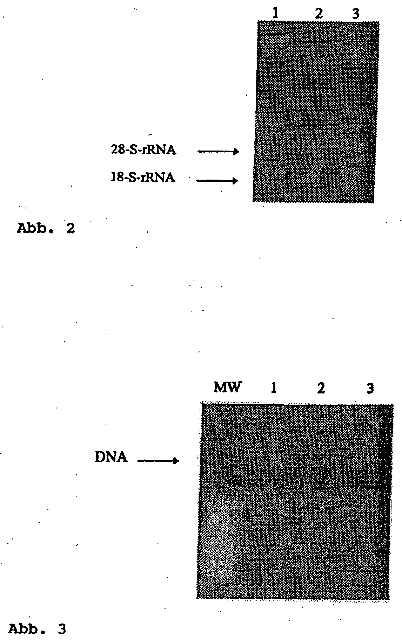

Für die Isolierung von RNA (Abb. 2) wurde der HighPure RNA Isolation Kit (Boehringer Mannheim, Kat.-Nr. 1828 665) verwendet. Die Beipackzettelvorschrift wurde folgendermaßen modifiziert: Ein Volumen von 2,4 ml Probenlysat wurde in 4 Aliquots mit jeweils 600 µl auf die Säule aufgetragen, so daß insgesamt Probenmaterial aus 2,4 ml Lysat aufgetragen wurden. Alle anderen Schritte wurden entsprechend dem Beipackzettel ausgeführt. Die RNA wurde schließlich mit 100 µl Elutionspuffer eluiert.

Zur Isolierung von DNA (Abb. 3) wurde der QiaAmp Blood Kit (Qiagen-Kat.-Nr. 29104) eingesetzt. Die im Beipackzettel beschriebene Standardprozedur wurde in verschiedenen Punkten modifiziert: 400 µl Probenvolumen wurden direkt auf die Säule gegeben, wobei das im Kit enthaltene Bindereagenz nicht eingesetzt wurde. 25 µl Proteinase-K-Stocklösung wurden zugefügt und die Probe 10 Min. bei Raumtemperatur inkubiert. Danach wurde die Säule in ein Sammelgefäß gestellt und wie im Beipackzettel beschrieben zentrifugiert. Alle weiteren Schritte wurden, bis auf die Verwendung von Ethanol, wie im Beipackzettel beschrieben durchgeführt. Das Elutionsvolumen war 200 µl.

### Beispiel 3:

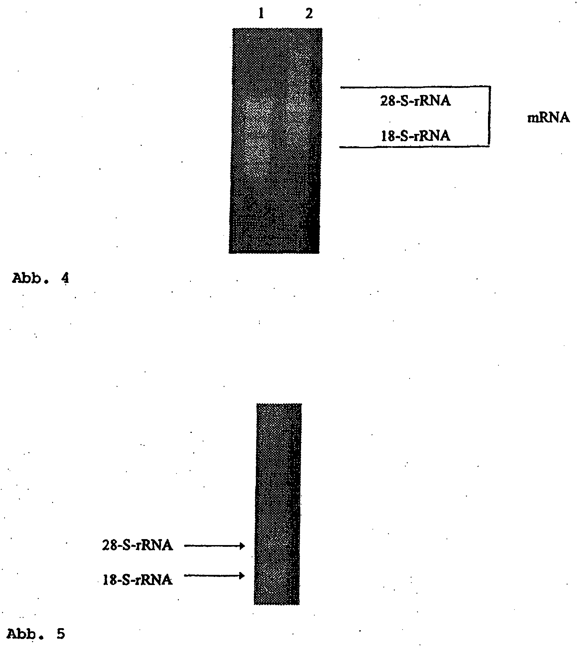

Isolierung von mRNA aus Probenlysat unter Verwendung von Streptavidinbeschichteten Magnetpartikeln und Biotin-markiertem Oligo(dT) (Abb. 4):

### Material und Methode:

20 ml Probenlysat wurden in ein Gefäß gegeben. Die mRNA wurde nach folgender Methode isoliert: Zunächst wurden 30 ml Hybridisierungspuffer (20 mM Tris-HCl, 300 mM NaCl. 6 nM Biotin-markiertes Oligo(dT), pH 7,4) zum Lysat gegeben. Danach wurden 3 mg Streptavidin-Magnetpartikel (Boehringer Mannheim) zugegeben. Die Probe wurde gemischt und für 5 min bei Raumtemperatur inkubiert. Die Magnetpartikel wurden mit Hilfe eines Magneten abgetrennt, der Überstand wurde verworfen. Danach wurden die Partikel in Waschpuffer 1 (10 mM Tris-HCl, 200 mM NaCl, 1% Triton-X-100, pH 7,5) resuspendiert und 3 mal mit Waschpuffer 2 (10 mM Tris-HCl, 200 mM NaCl, pH 7,5) gewaschen (Waschschritte: Resuspension, magnetische Separation, Entfernung des Überstandes). Nach dem letzten Waschschritt wurde der Überstand komplett entfernt und die Partikel wurden in 20 µl dest. Wasser resuspendiert. Die Probe wurde für 5 min. auf 70°C erhitzt. Die Magnetpartikel wurden separiert und der Überstand, der die mRNA enthielt, wurde mittels Gelelektrophorese analysiert.

### Beispiel 4:

Isolierung der DNA und RNA unter Verwendung einer abgeänderten Vorschrift nach Chomczynski und Sacchi (Analytical Biochemistry 162, 156-159 (1987)) (Beispiel für eine auf Lösungsmittelextraktion basierende Methode) (Abb. 5):

### Material und Methode:

2 ml Probenvolumen wurden aus dem Blutentnahmegefäß in ein Röhrchen überführt. Danach wurden 0,2 ml einer 2 M Natriumacetat-Lösung, pH 4, 2 ml Phenol (Wasser gesättigt) und 0,4 ml eines Chloroform-Isoamylalkohol-Gemisches (49:1) zugegeben, wobei nach Zugabe jeder Lösung die Probe gründlich gemischt wurde. Die komplette Lösung wurde 10 Sekunden heftig geschüttelt und 15 Minuten auf Eis inkubiert. Die Probe wurde 20 Minuten bei 4°C mit 10000 g zentrifugiert. Nach Zentrifugation befand sich die RNA in der wässrigen Phase, die DNA und Proteine in der Zwischen- und Phenolphase. Die wässrige Phase wurde in ein neues Gefäß überführt und mit 1 ml Isopropanol versetzt. Zur Ausfällung der RNA wurde die Probe für 1 Stunde bei -20°C gelagert. Nach erneuter Zentrifugation bei 4°C mit 10000 g befand sich die RNA im Pellet. Dieses wurde in 0,3 ml Puffer (4 M Guanidiniumthiocyanat, 25 mM Natriumcitrat, pH 7,0, 0,5% Sarcosyl, 0,1 M 2-Mercaptoisopropanol) aufgenommen, in ein neues 1,5 ml Eppendorf-Gefäß überführt und mit 1 Volumen Isopropanol versetzt. Nach 1 Stunde Inkubation bei -20°C wurde die Lösung in einer Eppendorf-Zentrifuge 10 Minuten bei 4°C zentrifugiert. Das RNA-Pellet wurde in 75% Ethanol aufgenommen und über Zentrifugation (Speed vac) eingeengt und getrocknet. Zur weiteren Verarbeitung wurde die Probe in 100 µl 10 mM Tris-HCl, pH 6,5 gelöst.

### Beispiel 5

Bedeutung von reduzierenden Reagenzien (z.B. DTT) in der Stabilisierungslösung für die Langzeitstabilisierung von RNA

### Material und Methode:

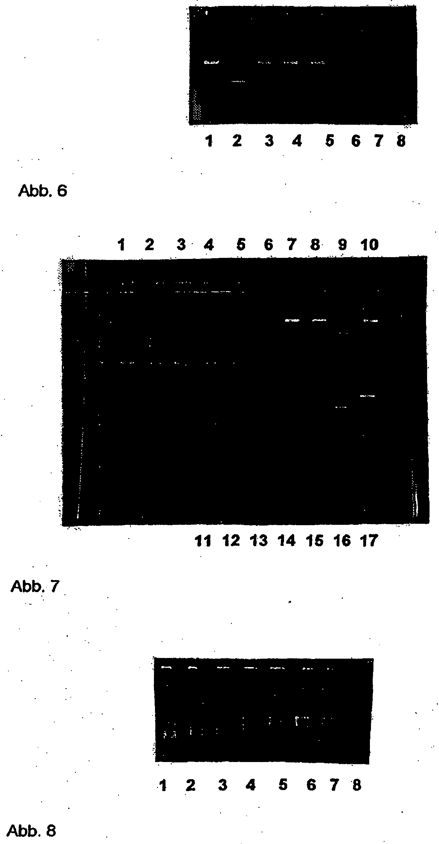

Verwendete Stabilisierungslösung: 4.0 M GTC; 13.5 % Triton X100; 45 mM Tris/HCl; mit bzw. ohne 120 mM DTT. 700 µl Serum wurden mit 700 µl Stabilisierungslösung gemischt. Nach 2 min Inkubation wurden 20 µl MS2-RNA (0.8 µg/µl von Roche) zugegeben. Die Proben wurden für 180 min bei 40 °C inkubiert und anschließend in Aliquots a 400 µl mit dem High Pure total RNA Kit von Roche aufgearbeitet. Die Proben wurden in einem Schritt ohne Zusatz des Kit-Bindereagenz auf die Säule gegeben und nach Anleitung zentrifugiert. Die folgenden Waschschritte und die Elution der RNA in 50 µl Elutionbuffer erfolgten nach Anleitung.
Die Analytik erfolgte mittels Agarosegel (siehe Abb. 6).
Ergebnis: Ohne den Zusatz von reduzierenden Reagenzien zur Stabilisierungslösung kann keine Langzeitstabilisierung von RNA erreicht werden.

### Beispiel 6

Stabilität von freier MS2-RNA in Serum. Kinetik des RNA-Abbaus durch Probenkomponenten.

### Material und Methode:

250 µl Serum wurden mit 10 µl MS2-RNA (0.8 µg/µl von Roche) gespiked und bei Raumtemperatur inkubiert. Sofort nach Zugabe der RNA, nach 2 min bis 50 min wurde der natürliche RNA-Abbau in Serum durch Zusatz von 250 µl Stabilisierungslösung abgestoppt. Alle Ansätze wurden als Doppelbestimmung durchgeführt. Als Standard wurde eine Probe erst nach Zusatz der Stabilisierungslösung zum Serum mit MS2-RNA versetzt und parallel aufgearbeitet.
Alle Proben wurden parallel mit dem High Pure viral RNA-Kit von Roche aufgearbeitet. Die Proben wurden in einem Schritt ohne Zusatz des Kit-Bindereagenz auf die Säule gegeben und nach Anleitung zentrifugiert. Die folgenden Waschschritte und die Elution der RNA in 50 µl Elutionspuffer erfolgten nach Anleitung. 20 µl des Eluates wurden mittels eines 1.2 %igen nativen Agarosegeles aufgetrennt und analysiert (siehe Abb. 7).

Ergebnis: MS2-RNA ist in Serum nicht stabil. Bereits 2 Minuten nach der Zugabe von RNA zu Serum ist diese vollständig abgebaut Durch den Zusatz von Stabilisierungslösung zum Serum im Verhältnis 1:1 kann dieser Prozeß sofort abgestoppt und eine Stabilisierung der RNA zum Zeitpunkt der Zugabe der Stabilisierungslösung (=Blutabnahme) erreicht werden.

### Beispiel 7

### Stabilität von MS2-RNA in Serum/Stabilisierungslögung: Abhängigkeit von derGTC Konzentration

### Material und Methode

Verwendete Stabilisierungslösungen: 3 - 5 M GTC; 13.5 % Triton X100; 50 mM DTT; 42 mM Tris/HCl
pH der Lösungen:ca.4.0
pH der Lösungen nach Serumzugabe: ca. 6.7.

2 ml Serum wurden mit 2.5 ml der jeweiligen Stabilisierungslösungen gemischt. Nach einer Inkubationszeit von 2-5 min wurden 90 µl MS2-RNA (0.8 µg/µl von Roche) zugegeben und bei 40 °C inkubiert. In regelmäßigen Abständen wurden 400 µl Probe entnommen und mit dem High Pure total RNA Kit von Roche entsprechend Beispiel 5 aufgearbeitet. Die Proben wurden in 50 µl eluiert und bei - 20 °C eingefroren. Für die Analyse der RNA-Integrität wurden 20 µl des Eluates auf ein 1.5 %iges Agarosegel aufgetragen (s. Abb. 8).
Für die PCR-Analyse der Proben wurden 10 µl des Eluats mittels AMV-RT (Roche) reverse transkribiert und anschließend mittels PCR auf dem Lightcycler analysiert:

| | | |
|---|---|---|
| Ansatz für RT: | 4.0 µl | AMV-RT-Puffer |
| (42 °C für 1 h) | 2.0 µl | dNTP's ( Endkonzentration 10 mM) |
| | 0.5 µl | RNaseinhibitor (Roche, 20 units) |
| | 1.0 µl | Primer 2827 (Endkonzentration 1 µM) |
| | 1.9 µl | DMPC-Wasser |
| | 0.6 µl | AMV-RT (Roche, 15 units) |

| | |
|---|---|
| 10 µl | Template-RNA |
| 20 µl | |

Die PCR wurde auf dem Lightcycler bei einer Annealingtemperatur von 61 °C unter Verwendung von SYBR-Green als Detektionssystem durchgeführt. Ansatz für PCR:

| | | |
|---|---|---|
| 1.6 µl | MgCl₂ (Stammlsg. 25 mM) | |
| | 5.9 µl | DMPC-Wasser |
| | 0.25 µl | Primer 2827 (Stammlsg. 20 mM) |
| | 0.25 µl | Primer 2335 (Stammlsg. 20 mM) |
| | 1.0 µl | SYBR-Green-Mastermix (Roche) |
| | 1.0 µl | RT-Ansatz (1:50 verdünnt) |
| | 10 µl | |

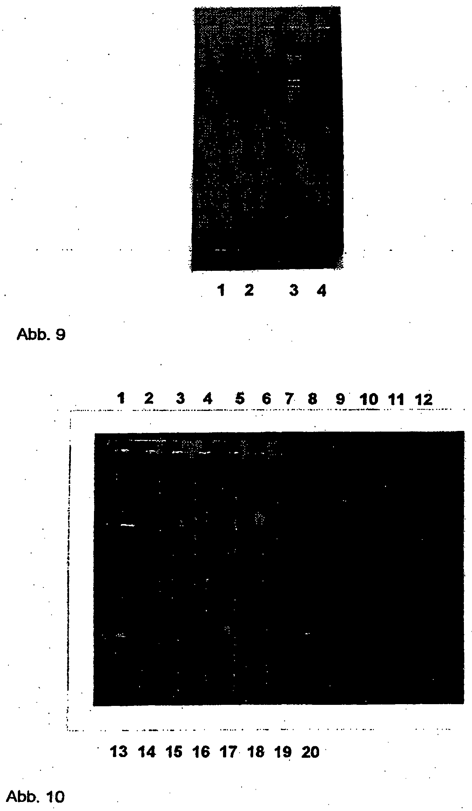

Das Amplifikat der PCR wurde vollständig auf ein 2 %iges Agarosegel aufgetragen (siehe Abb. 9).

### Ergebnis:

RNA-Integrität nach 3 Tagen bei 40 °C:
Das Agarosegel in Abb. 8 zeigt 20 µl der eluierten MS2-RNA nach 3 Tagen Inkubation bei 40 °C. Nach diesem Zeitraum sind in Abhängigkeit vom GTC-Gehalt deutliche Unterschiede in der RNA-Integrität zu erkennen. Demnach ist ein Salzgehalt kleiner 2 M in der Serum/Stabilisierungslösung für die Integrität der RNA von Vorteil.

Amplifizierbarkeit der RNA nach 8 Tagen bei 40 °C:
Obwohl bereits nach 3 Tagen bei 40 °C eine beginnende Degradierung der RNA festgestellt wurde, konnten alle RNA-Proben nach einer Inkubation von 8 Tagen bei 40 °C amplifiziert und eindeutig nachgewiesen werden.

Das Amplifikat der PCR wurde vollständig auf ein 2 %iges Agarosegel aufgetragen (siehe Abb. 9).

### Beispiel 8

Stabilität von MS2-RNA in Serum/Stabilisierungslösung: Abhängigkeit vom pH-Wert der mit Stabilisierungslösung versetzten Probe.

### Material und Methode

| | | |
|---|---|---|
| Verwendete Lösung: | 4 M (5 M) | GTC |
| | 14.4% | Triton X 100 |
| | 50 mM | DTT |
| | 45 mM | Tris/HCl |

pH nach Serumzugabe zwischen 6.7 und 8.0

2.5 ml Stabilisierungslösung wurden mit 2.0 ml Serum gemischt. Nach Zusatz von 90 µl MS2-RNA (0.8 µg/µl, Roche) wurden die Proben bei Raumtemperatur inkubiert. In regelmäßigen Abständen wurde die RNA aus 500 µl Probe mit dem Roche viral RNA Kit entsprechend Bsp. 6 aufgearbeitet und in 50 µl Elutionspuffer isoliert. 20 µl des Eluates wurden mittels Agarosegel analysiert (siehe Abb. 10).

Ergebnis:
Der pH der Serum/Stabilisierungslösung und damit auch der pH und Pufferbereich der Stabilisierungslösung ist für die Langzeitstabilisierung von RNA entscheidend. Während bei einem pH-Wert von 8.0 bereits nach 2 Tagen keine intakte RNA mehr nachgewiesen werden konnte, ist in einem pH-Bereich zwischen 6.6 und 7.0 noch nach 13 Tagen Inkubation bei Raumtemperatur intakte RNA nachweisbar.

Neben dem pH-Wert ist jedoch auch eine optimal eingestellte GTC-Konzentration für die Langzeitstabilisierung von RNA von Bedeutung (siehe auch Bsp. 7). Das dargestellte Bsp. verdeutlicht, dass fuer eine Langzeitstabilisierung von RNA eine GTC-Endkonzentration in der stabilisierten Probe von 2.2 M GTC besser ist als 2.78.

### Legenden

### Abb. 1:

Probenentnahmegefäß mit N-sS, definiertem Vacuum, mit Septum versiegelt.

### Abb. 2:

Gelanalyse (1% Agarose) von RNA, die im Probeentnahmegefäß unterschiedlich lange gelagert wurde. Spalte 1: Isolierung unmittelbar nach Probenentnahme (keine Lagerung), Spalte 2: Lagerung für einen Monat bei -20°C, Spalte 3: Lagerung für 6 Tage bei 4°C. Die Menge der aufgetragenen RNA entsprach einem Blutvolumen von 120 µl.

### Abb 3:

Gelanalyse (1% Agarose) von DNA, die im Probeentnahmegefäß unterschiedlich lange gelagert wurde. Spalte 1: Isolierung unmittelbar nach Probenentnahme (keine Lagerung), Spalte 2: Lagerung für einen Monat bei -20°C, Spalte 3: Lagerung für 6 Tage bei 4°C. Die Menge der aufgetragenen DNA entsprach einem Blutvolumen von 10 µl.

### Abb. 4:

Gelanalyse (1% Agarose) von mRNA, die aus 10 ml Blut isoliert wurde (Spalte 2). Molekulargewichtsmarker (Spalte 1). Zusätzlich zur mRNA sind die rRNA Banden sichtbar. Die scharfen Konturen der Banden beweisen die Integrität der Nukleinsäuren.

### Abb. 5:

Gelanalyse (1% Agarose) der RNA, die aus 120 µl Blut isoliert wurde.

### Abb. 6:

Gelanalyse von isolierter MS2-RNA nach Inkubation in Serum/Stabilisierungslösung mit/ohne. DTT für 180 min bei 40°C.

Spalte **1:** Positivkontrolle: MS2-RNA, Spalte 2: DNA-Marker, Spalte **3,4,5**: MS2-RNA nach Inkubation mit DTThaltiger Stabilisierungslösung, Spalte **6,7,8**: MS2-RNA nach Inkubation mit Stabilisierungslösung ohne DTT

### Abb. 7:

Gelanalyse von isolierter MS2-RNA nach Inkubation in Serum für 0-50 min

Spalte **10,17**: MS2-RNA-Standard, Spalte **9,16**: DNA-Marker, Spalte **7,8**: Inkubation für 0 min, Spalte **5,6**: Inkubation für 2 min, Spalte **3,4**: Inkubation für 5 min, Spalte **1,2**: Inkubation für 10 min, Spalte **11,12**: Inkubation für 15 min, Spalte **13,14**: Inkubation für 30 min, Spalte 15: Inkubation für 50 min

### Abb. 8:

Gelanalyse von MS2-RNA, die nach Inkubation in Serum/Stabilisierungslösung für 3 Tage bei 40 °C isoliert wurde. Der GTC-Gehalt der Stabilisierungslösung nach Serumzugabe, in welcher die betreffende RNA-Probe inkubiert wurde,
ist in der entsprechenden Spalte angegeben.

Spalte 1: 2.70 M GTC, Spalte **2**:2.5 M GTC, Spalte **3:** 2.36 M GTC, Spalte **4**:2.20 M GTC, Spalte **5**: 2.08 M GTC, Spalte **6:** 1.94 M GTC, Spalte **7:** 1.80 M GTC, Spalte **8:** 1.66 M GTC.

### Abb. 9:

Gelanalyse der PCR-Amplifikate von MS2-RNA,welche nach 1 bzw. 8 Tagen Inkubation bei 40 °C in Serum/Stabilisierungslösung isoliert wurde.

Spalte **1**: Amplifikat der nach 1 Tag isolierten RNA, Spalte **2**: Amplifikat der nach 8 Tagen isolierten RNA, Spalte **3**: DNA-Marker, Spalte **4**: MS2-RNA-Positivkontrolle: 0.8 µg in 10 µl RT, 1:50 verdünnt, 1 µl amplifiziert

### Abb.10:

Gelanalyse von isolierter MS2-RNA nach 6 (Spalte **2-12**) bzw. 13 (Spalte **14-19**) Tagen Inkubation bei Raumtemperatur in Serum/Stabilisierungslösung. Hinter den betreffenden Spalten steht der pH-Wert, welcher nach Mischung von Serum und Stabilisierunslösung errreicht wurde.

Spalte **1**, **13, 20**: DNA-Marker, Spalte **2**: pH 8.0, Spalte **3**: pH 7.7, Spalte 4:pH 7.5, Spalte 5:pH 7.35, Spalte **6**: pH 7.18, Spalte **7,14**:pH 7.07, Spalte **8,15**: pH 6.94, Spalte **9,16**: pH 6.8, Spalte **10,17**: pH 6.72, Spalte **11,18**: pH 6.68 und Spalte **12,19**: pH 6.7 Die Stabilisierunslösung der RNA in Spalte **12, 19** hatte den gleichen pH-Wert wie die der RNA in Spalte **11**, enthielt aber 5 M GTC anstelle von 4 M.

## Patentansprüche

1. Gefäß zur Blutentnahme, welches im zur Blutaufnahme vorgesehenen Raum einen Unterdruck aufweist , enthaltend eine wässrige Lösung mit folgenden Bestandteilen:
- ein Guanidiniumsalz;
- eine Puffersubstanz und
- ein Detergenz.

2. Gefäß nach Anspruch 1, **dadurch gekennzeichnet, dass** das Guanidiniumsalz ausgewählt wird aus Guanidiniumthiocyanat und Guanidiniumchlorid.

3. Gefäß nach einem der Ansprüche 1 bis 2, **dadurch gekennzeichnet, dass** das Guanidiniumsalz in einer Konzentration von 1 bis 8,0 M, vorzugsweise 2,5 bis 8,0 M vorliegt.

4. Gefäß nach einem der Ansprüche 1 bis 3, **dadurch gekennzeichnet, dass** die Puffersubstanz ausgewählt wird aus Tris, HEPES, MOPS, Citrat- und Phosphatpuffer.

5. Gefäß nach einem der Ansprüche 1 bis 4, **dadurch gekennzeichnet, dass** die Puffersubstanz in einer Konzentration von 10 bis 300 mM vorliegt.

6. Gefäß nach einem der Ansprüche 1 bis 5, **dadurch gekennzeichnet, dass** das Detergenz ausgewählt wird aus Triton-X-100®, NP-40®, Polydocanol und Tween 20®.

7. Gefäß nach einem der Ansprüche 1 bis 6, **dadurch gekennzeichnet, dass** das Detergenz in einer Konzentration von 5 bis 30 Gew. % vorliegt.

8. Gefäß nach einem der Ansprüche 1 bis 7, **dadurch gekennzeichnet, dass** das Gefäß zusätzlich ein Reduktionsmittel enthält.

9. Gefäß nach einem der Ansprüche 1 bis 8, **dadurch gekennzeichnet, dass** das Reduktionsmittel ausgewählt wird aus Dithiothreitol, 3-Mercaptoethanol und TCEP.

10. Gefäß nach einem der Ansprüche 1 bis 9, **dadurch gekennzeichnet, dass** das Reduktionsmittel in einer Konzentration von 0,1 bis 10,0 Gew. % vorliegt.

11. Gefäß nach einem der Ansprüche 1 bis 10, **dadurch gekennzeichnet, dass** der pH der Lösung zwischen 4,0 und 7,5 oder zwischen 4,0 und 6,5 liegt.

12. Gefäß nach einem der Ansprüche 1 bis 11, **dadurch gekennzeichnet, dass** es entnommenes Blut enthält.

13. Gefäß nach Anspruch 12, **dadurch gekennzeichnet, dass** die darin enthaltene Blutmenge dem 0,1- bis 4-fachen Volumen der Lösung in dem Gefäß entspricht.

14. Gefäß nach Anspruch 13, **dadurch gekennzeichnet, dass** die Endkonzentration des Guanidiniumsalzes nach Blutzufuhr zwischen 1,0 M und 5 M oder zwischen 1,5 M und 5 M liegt.

15. Verfahren zur Stabilisierung und/oder Isolierung von Nukleinsäuren aus Blut, **dadurch gekennzeichnet, dass** sich das Blut direkt nach Entnahme in einem Gefäß nach einem der Ansprüche 1 bis 14 befindet und gegebenenfalls umfassend die Isolierung der Nukleinsäuren mit herkömmlichen Verfahren.

16. Verfahren nach Anspruch 15, **dadurch gekennzeichnet, dass** der pH-Wert der Lösung so eingestellt ist, dass nach Zugabe des Probenmaterials ein pH-Wert zwischen 4,0 und 7,5 erreicht wird.

## Claims

1. Blood withdrawing vessel having an evacuated chamber which is provided for receiving blood, the vessel comprising an aqueous solution with the following components:
- a guanidinium salt,
- a buffering agent, and
- a detergent.

2. Vessel according to claim 1, **characterised in that** the guanidinium salt is selected from guanidinium thiocyanate and guanidinium chloride.

3. Vessel according to any of claims 1 to 2, **characterised in that** the guanidinium salt is present in a concentration of from 1 to 8.0 M, preferably 2.5 to 8.0 M.

4. Vessel according to any of claims 1 to 3, **characterised in that** the buffering agent is selected from Tris, HEPES, MOPS, citrate and phosphate buffer.

5. Vessel according to any of claims 1 to 4, **characterised in that** the buffering agent is present in a concentration of from 10 to 300 mM.

6. Vessel according to any of claims 1 to 5, **characterised in that** the detergent is selected from Triton-X-100®, NP-40®, Polydocanol and Tween 20®.

7. Vessel according to any of claims 1 to 6, **characterised in that** the detergent is present in a concentration of from 5 to 30 % (w/w).

8. Vessel according to any of claims 1 to 7, **characterised in that** the vessel additionally comprises a reducing agent.

9. Vessel according to any of claims 1 to 8, **characterised in that** the reducing agent is selected from dithiothreitol, 3-Mercaptoethanol and TCEP.

10. Vessel according to any of claims 1 to 9, **characterised in that** the reducing agent is present in a concentration of from 0.1 to 10.0 % (w/w).

11. Vessel according to any of claims 1 to 10, **characterised in that** the pH of the solution is between 4.0 and 7.5 or between 4.0 and 6.5.

12. Vessel according to any of claims 1 to 11, **characterised in that** it comprises collected blood.

13. Vessel according to claim 12, **characterised in that** the amount of blood contained therein corresponds to 0.1 to 4 times the volume of the solution in the vessel.

14. Vessel according to claim 13, **characterised in that** the final concentration of guanidinium salt after addition of blood is between 1.0 M and 5 M or between 1.5 M and 5 M.

15. Method for the stabilization and/or isolation of nucleic acids from blood, **characterised in that** the blood is present, directly after collection, in a vessel according to any one of claims 1 to 14, optionally comprising the isolation of nucleic acids using established methods.

16. Method according to claim 15, **characterised in that** the pH of the solution is adjusted such that it has a pH of between 4.0 and 7.5 upon addition of the sample material.

## Revendications

1. Récipient destiné à un prélèvement sanguin, qui présente une pression négative dans la chambre prévue pour recueillir le sang et qui contient une solution aqueuse comprenant les ingrédients suivants:
- un sel de guanidinium ;
- une substance tampon et
- un détergent.

2. Récipient selon la revendication 1, **caractérisé en ce que** le sel de guanidinium est choisi parmi le thiocyanate de guanidinium et le chlorure de guanidinium.

3. Récipient selon l'une des revendications 1 à 2, **caractérisé en ce que** le sel de guanidinium est présent en une concentration de 1 à 8,0 M, de préférence 2,5 à 8,0 M.

4. Récipient selon l'une des revendications 1 à 3, **caractérisé en ce que** la substance tampon est choisie parmi Tris, HEPES, MOPS, le tampon citrate et le tampon phosphate.

5. Récipient selon l'une des revendications 1 à 4, **caractérisé en ce que** la substance tampon est présente en une concentration de 10 à 300 mM.

6. Récipient selon l'une des revendications 1 à 5, **caractérisé en ce que** le détergent est choisi parmi Triton-X-100®, NP-40®, Polydocanol et Tween 20®.

7. Récipient selon l'une des revendications 1 à 6, **caractérisé en ce que** le détergent est présent en une concentration de 5 à 30 % en poids.

8. Récipient selon l'une des revendications 1 à 7, **caractérisé en ce que** le récipient contient en plus un agent réducteur.

9. Récipient selon l'une des revendications 1 à 8, **caractérisé en ce que** l'agent réducteur est choisi parmi le dithiothréitol, le 3-mercaptoéthanol et le TCEP.

10. Récipient selon l'une des revendications 1 à 9, **caractérisé en ce que** l'agent réducteur est présent en une concentration de 0,1 à 10,0 % en poids.

11. Récipient selon l'une des revendications 1 à 10, **caractérisé en ce que** le pH de la solution est situé entre 4,0 et 7,5 ou entre 4,0 et 6,5.

12. Récipient selon l'une des revendications 1 à 11, **caractérisé en ce qu'**il contient du sang prélevé.

13. Récipient selon la revendication 12, **caractérisé en ce que** la quantité de sang qu'il contient correspond à 0,1 à 4 fois le volume de la solution dans le récipient.

14. Récipient selon la revendication 13, **caractérisé en ce que** la concentration finale en sel de guanidinium après apport du sang se situe entre 1,0 M et 5 M ou entre 1,5 M et 5 M.

15. Procédé pour la stabilisation et/ou l'isolation des acides nucléiques à partir du sang, **caractérisé en ce que** le sang se trouve directement après son prélèvement dans un récipient selon l'une des revendications 1 à 14, et comprenant le cas échéant l'isolation des acides nucléiques par des procédés traditionnels.

16. Procédé selon la revendication 15, **caractérisé en ce que** le pH de la solution est ajusté pour obtenir un pH situé entre 4,0 et 7,5, après addition de la matière d'échantillon.
